(19) Europäisches Patentamt
European Patent Office
Office européen des brevets

(11) Veröffentlichungsnummer: **0 352 675**
**A2**

# (12) EUROPÄISCHE PATENTANMELDUNG

(21) Anmeldenummer: **89113503.0**

(22) Anmeldetag: **22.07.89**

(51) Int. Cl.⁴: **C07D 405/06 , C07D 233/92 , C07D 233/68 , C07D 233/90 , C07D 233/56 , C07D 405/14 , C07D 401/06 , C07D 401/14 , A01N 43/50**

(30) Priorität: **28.07.88 DE 3825586**

(43) Veröffentlichungstag der Anmeldung:
**31.01.90 Patentblatt 90/05**

(84) Benannte Vertragsstaaten:
**AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Karbach, Stefan, Dr.**
**Grundwiesenweg 44**
**D-6730 Neustadt(DE)**
Erfinder: **Goetz, Norbert, Dr.**
**Schoefferstrasse 25**
**D-6520 Worms 1(DE)**
Erfinder: **Sauter, Hubert, Dr.**
**Neckarpromenade 20**
**D-6800 Mannheim 1(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

## (54) Substituierte Imidazolylmethyloxirane und substituierte Imidazolylpropene, ihre Herstellung und sie enthaltende Fungizide.

(57) Substituierte Imidazolylmethyloxirane und substituierte Imidazolylpropene der allgemeinen Formel

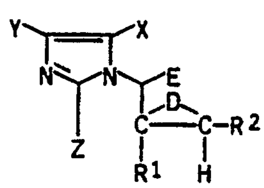

I

in welcher
$R^1$ und $R^2$ Alkyl, Cycloalkyl, Cycloalkenyl, Tetrahydropyranyl, Norbornyl, Pyridyl, Naphthyl, Biphenyl oder Phenyl bedeuten,
D den Rest 0 oder eine Einfachbindung bedeutet,
E den Rest H, F, Cl oder Br bedeutet,
X, Y und Z Wasserstoff, Halogen, Nitro, Cyano, Alkyl, Halogenalkyl, Alkoxy, Amino, gegebenenfalls durch substituiertes Amino, Mercapto, gegebenenfalls substituiertes Mercapto, Acyl, Alkoxycarbonyl, Hydroxyalkyl und gegebenenfalls substituiertes Phenyl bedeuten,
sowie deren für Pflanzen verträgliche Säureadditionssalze und Metallkomplexe, Verfahren zu ihrer Herstellung und sie enthaltende Fungizide.

EP 0 352 675 A2

## Substituierte Imidazolylmethyloxirane und substituierte Imidazolylpropene, ihre Herstellung und sie enthaltende Fungizide

Die vorliegende Erfindung betrifft neue substituierte Imidazolylmethyloxirane und substituierte Imidazolylpropene, Verfahren zu ihrer Herstellung und sie enthaltende Fungizide sowie Verfahren zur Bekämpfung von Pilzen.

Es ist bekannt, Imidazolyloxirane, z.B. das cis-2-(imidazol-1-yl-methyl)-2-(4-fluorphenyl)-3-(2,6-difluorphenyl)-oxiran (DE-32 18 130) als Fungizide zu verwenden. Die fungiziden Wirkungen sind jedoch unbefriedigend.

Es wurde nun gefunden, daß substituierte Imidazolylmethyloxirane und substituierte Imidazolylpropene der Formel I

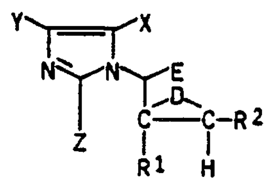

I

in welcher

$R^1$ und $R^2$ gleich oder verschieden sind und $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4-C-Atomen substituierte sein können,

D den Rest 0 oder eine Einfachbindung bedeutet,

E den Rest H, F, Cl oder Br bedeutet,

X, Y und Z gleich oder verschieden sind und Wasserstoff, Halogen, Nitro, Cyano, $C_1$-$C_3$-Alkyl, $C_1$-$C_3$-Halogenalkyl, $C_1$-$C_3$-Alkoxy, Amino, gegebenenfalls durch $C_1$-$C_3$-Alkyl, $C_1$-$C_3$-Acyl substituiertes Amino, Mercapto, gegeAlkoxycarbonyl $C_1$-$C_3$-Alkyl substituiertes Mercapto, $C_1$-$C_3$-Acyl, $C_1$-$C_3$-benenfalls durch $C_1$-$C_3$-Hydroxyalkyl oder gegebenenfalls substituiertes Phenyl bedeuten, wobei X, Y und Z nicht gleichzeitig Wasserstoff bedeuten, sowie deren für Pflanzen verträgliche Säureadditionssalze und Metallkomplexe eine bessere fungizide Wirkung besitzen als bekannte Azolverbindungen.

Die Verbindungen der Formel I enthalten asymmetrische C-Atome und können daher als Enantiomere und Diastereomere auftreten. Die Gemische von Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base trennen. Als fungizide Mittel kann man sowohl die einheitlichen Diastereomere bzw. Enantiomere als auch deren bei der Synthese anfallenden Gemische verwenden.

$R^1$ bzw. $R^2$ bedeuten beispielsweise $C_1$-$C_8$-Alkyl, insbesondere $C_1$-$C_4$-Alkyl, (Methyl, Ethyl, Isopropyl, n-Propyl, n-Butyl, sec-Butyl, tert.-Butyl, n-Pentyl, Neopentyl), 1-Naphthyl, 2-Naphthyl, p-Biphenyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, $C_1$-$C_4$-Alkoxyphenyl, 2-Methoxyphenyl, 3-Methoxyphenyl, 4-Methoxyphenyl, 2,4-Dimethoxyphenyl, $C_1$-$C_4$-Alkylphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Aminophenyl, 2-Trifluormethylphenyl, 3-Trifluormethylphenyl, 4-Trifluormethylphenyl, 3-Pyridyl, Tetrahydropyranyl, Cyclopropyl, Cyclopentyl, Cyclohexyl, 2-Cyclohexenyl, 3-Cyclohexenyl, Norbornyl.

X, Y and Z bedeuten beispielsweise Wasserstoff, Methyl, Ethyl, Propyl, Isopropyl, Trifluormethyl, Methoxy, Ethoxy, Methylamino, Dimethylamino, Acetylamino, Diacetylamino, Methylthio, Acetyl, Methoxyacetyl, Phenyl, Halogenphenyl, Chlorphenyl, Hydroxyethyl, wobei X, Y and Z nicht gleichzeitig Wasserstoff bedeuten, d.h. mindestens einer der Reste X, Y oder Z verschieden von Wasserstoff ist.

Säureadditionssalze sind beispielsweise die Hydrochloride, Bromide, Sulfate, Nitrate, Phosphate, Oxalate, oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der

Imidazolylmethyloxirane (I) mit den Säuren.

Metallkomplexe der Wirkstoffe I oder ihrer Salze können z.B. mit Kupfer, Zink, Zinn, Mangan, Eisen, Kobalt oder Nickel gebildet werden, indem man die Imidazolylmethyloxirane mit entsprechenden Metallsalzen umsetzt, z.B. mit Kupfersulfat, Zinkchlorid, Zinnchlorid, Mangansulfat.

Die Verbindungen der Formel I, in denen D den Rest O und E den Rest H bedeutet, können z.B. hergestellt werden, indem man

a) eine Verbindung der Formel II

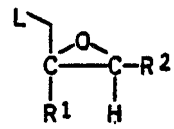

II

in welcher $R^1$ und $R^2$ die oben angegebenen Bedeutungen haben und L eine nukleophil substituierbare Abgangsgruppe (z.B. Halogen, OH) bedeutet, mit einer Verbindung der Formel III

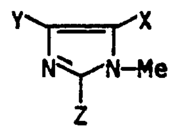

III

in der Me ein Wasserstoffatom, ein Metallatom (z.B. Na, K) oder eine Trimethylsilylgruppe bedeutet und X, Y und Z die oben angegebene Bedeutung hat, zur Umsetzung bringt, oder

b) eine Verbindung der Formel IV

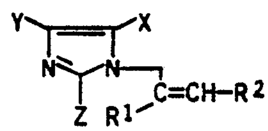

IV

in welcher $R^1$, $R^2$, X, Y und Z die oben angegebene Bedeutung haben, in das Epoxid überführt.

Die Reaktion a) erfolgt z.B. - falls Me ein Wasserstoffatom bedeutet -gegebenenfalls in Gegenwart einer Base, gegebenenfalls in Gegenwart eines Lösungs- oder Verdünnungsmittels, gegebenenfalls unter Zusatz eines Reaktionsbeschleunigers bei Temperaturen zwischen 10 und 120°C. Zu den bevor zugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Cäsiumcarbonat oder Natrium-, Kalium- oder Cäsiumhydrogencarbonat, Pyridin oder 4-Dimethylaminopyridin. Es können aber auch andere übliche Basen verwendet werden.

Als Reaktionsbebschleuniger kommen vorzugsweise Metallhalogenide wie Natriumjodid, oder Kaliumjodid, quaternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen z.B. bei Temperaturen zwischen 20 und 150°C, drucklos oder unter Druck, kontinuierlich oder diskontinuierlich durchgeführt.

Ist Me ein Metallatom wird die Reaktion a) z.B. gegebenenfalls in Gegenwart eines Lösungs- oder Verdünnungsmittels und gegebenenfalls unter Zusatz einer starken anorganischen oder organischen Base bei Temperaturen zwischen -10 und 120°C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie

Natrium- und Kaliumamid, ferner Natrium- oder Kalium-tert.-butoxid, Lithium-, Natrium- oder Kaliumtriphenylmethyl und Naphthalinlithium, -natrium oder -kalium.

Für die Reaktion b) kommen z.B. als Verdünnungsmittel polare organische Lösungsmittel wie Nitrile, z.B. Acetonitril, Sulfoxide, z.B. Dimethylsulf oxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen z.B. zwischen 0 und 100°C, vorzugsweise bei 20 bis 80°C. Bei Anwesenheit eines Lösungsmittels wird zweckmäßig beim Siedepunkt des jeweiligen Lösungsmittels gearbeitet.

Die neuen Ausgangsverbindungen II erhält man z.B. durch Epoxidierung der entsprechenden Olefine V:

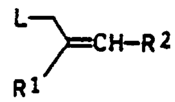

V

(vgl. G. Dittus in Houben-Weyl-Müller, Methoden der Organischen Chemie, Georg Thieme Verlag, Stuttgart, 1965, Bd. VI, 3, Seite 385 ff).

Die Verbindung V stellt man her, indem man Olefin der Formel IX

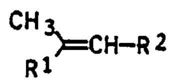

IX

nach bekannten Methoden in Allylposition halogeniert oder oxidiert.

Geeignete Halogenierungsreagenzien sind N-Chlor- und N-Bromsuccinimid in halogenierten Kohlenwasserstoffen wie Tetrachlorkohlenstoff, Trichlorethan oder Methylenchlorid bei Temperaturen zwischen 20 und 100°C. Zur Allyloxidation verwendet man Perester wie Perbenzoesäure-tert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100°C.

Die so erhaltenen Allylhalogenide bzw. -alkohole V werden anschließend in die entsprechenden Epoxide II (L = Halogen, OH) übergeführt. Dazu oxidiert man die Olefine V mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatiumhydrogenphosphat, Triton B. Man arbeitet zwischen 10 und 100°C und katalysiert die Reaktion gegebenenfalls z.B. mit Jod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30°C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Während die so erhaltenen Epoxihalogenide II (L = Halogen) gemäß Verfahren a) sofort umgesetzt werden können, überführt man die entsprechenden Epoxialkohole II (L = OH) z.B. in reaktive Ester, die dann mit den Verbindungen III gemäß Verfahren a) umgesetzt werden.

Die Darstellung der reaktiven Ester, die mit III umgesetzt werden können, erfolgt nach allgemein bekannten Methoden (Houben-Weyl-Müller, Methoden der organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Verbindungen V lassen sich entsprechend allgemein bekannten Verfahren zur Olefinsynthese (Houben-Weyl-Müller, Methoden der organischen Chemie, Georg Thieme Verlag, Stuttgart, 1972 Bd. V, 1b) herstellen.

Die Verbindungen der Formel IV

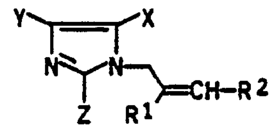

IV

werden z.B. erhalten, indem man

a) eine Verbindung der Formel V

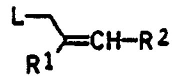

V

in welcher $R^1$ und $R^2$ die oben angegebene Bedeutung haben und L eine nukleophil substituierbare Abgangsgruppe bedeutet, mit einer Verbindung der Formel III

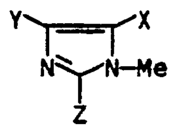

III

in der Me ein Wasserstoffatom, ein Metallatom oder eine Trimethylsilylgruppe bedeutet und X, Y und Z die oben angegebene Bedeutung hat, zur Umsetztung bringt, oder

b) eine Verbindung der Formel VI

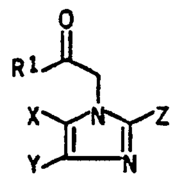

VI

in welcher $R^1$, X, Y und Z die oben angegebene Bedeutung hat, mit einer Verbindung der Formel VII

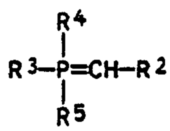

VII

in der $R^2$ die gleiche Bedeutung wie in Formel I hat und $R^3$, $R^4$ und $R^5$, die gleich oder verschieden sein können, den Phenylrest, den p-Carboxyphenylrest, den p-Dimethylaminophenylrest, die Dimethylamino, Piperidino- oder Morpholinogruppe, Alkylreste mit 1 bis 3-C-Atomen oder die Cyclohexylgruppe darstellen, zur Umsetzung bringt.

Die Verbindungen der Formel I, in denen E den Rest F, Cl oder Br bedeutet, können z.B. hergestellt werden, indem man eine Verbindung der Formel VIII

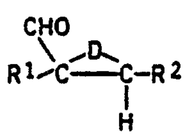

VIII

in welcher $R^1$, $R^2$ und D die angegebenen Bedeutungen haben, mit einer Verbindung der Formel III

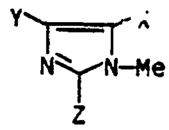

III

in der X, Y, Z und Me die oben genannten Bedeutungen haben, in Gegenwart eines Thionylhalogenids zur Umsetzung bringt.

Die Umsetztung erfolgt z.B. gegebenenfalls in Gegenwart eines Lösungs- oder Verdünnungsmittels bei Temperaturen zwischen -30 und 80°C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die neuen Ausgangsverbindungen VIII, in denen D den Rest O bedeutet, erhält man z.B. durch Epoxidierung der entsprechenden Olefine X

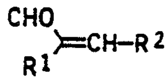

X

(vgl. G. Dittus in Houben-Weyl-Müller, Methoden der Organischen Chemie, Georg Thieme Verlag, Stuttgart, 1965, Bd VI, 3, Seite 38 ff).

Die Verbindungen X lassen sich entsprechend allgemein bekannten Verfahren zur Aldehydsynthese (Houben-Weyl-Müller, Methoden der Organischen Chemie, Georg Thieme Verlag, Stuttgart 1983, Bd E3) herstellen.

Die folgenden Beispiele erläutern die Herstellung der Wirkstoffe.

I. Herstellung der Ausgangsstoffe

Beispiel A

Zu einer Lösung von 85,5 g 2-Trifluormethylbenzaldehyd in 300 ml Methanol werden 8,4 g Natriumhydroxid in 40 ml Wasser gegeben. Das Reaktionsgemisch wird auf 10°C gekühlt und schnell 69 g 4-Fluorphenylacetaldehyd zugetropft, wobei die Temperatur in der Lösung 30°O nicht übersteigt. Nach zweistündigem Rühren bei Raumtemperatur wird der farblosen Reaktionslösung 300 ml Wasser zugesetzt und die entstandene Emulsion mit Methyl-tert.- butylether ausgeschüttelt. Die organische Phase wird abgetrennt, über Natriumsulfat getrocknet und im Vakuum eingedampft. Bei der anschließenden Destillation des verbleibenden Rückstandes werden bei 0,3 mbar und 116°C Übergangstemperatur 96 g (65 %) E-2-(4-Fluorphenyl)-3-(2-trifluormethylphenyl)-propenal erhalten.

Beispiel B

96 g E-2-(4-Fluorphenyl)-3-(2-trifluormethylphenyl)-propenal werden in 300 ml Methanol gelöst und 2,3 ml Natronlauge (konz.) zugesetzt. Die Reaktionslösung wird bei 0°C gerührt, während 27,7 g Wasserstoffperoxyd (ca. 50 %ig) langsam zugetropft werden, wobei die Innentemperatur von 30°C nicht überschritten wird. Nach beendeter Zugabe wird sechs Stunden bei Raumtemperatur (20°C) gerührt und anschließend 5 g Natriumborhydrid zugegeben, das in wenig 10 %iger Natronlauge gelöst war. Nachdem das Reaktionsgemisch 18 Stunden bei Raumtemperatur rührte, wird der Lösung 100 ml Wasser zugesetzt und die entstandene Emulsion mit Methylenchlorid ausgeschüttelt. Die isolierte organische Phase wird daraufhin über Natriumsulfat getrocknet und eingeengt. Man erhält 90 g (89 %) cis-2-Hydroxymethyl-2-(4-fluorphenyl)-3-(2-trifluormethylphenyl)-oxiran.

Beispiel C

Zu einer Lösung von 90 g cis-2-Hydroxymethyl-2-(4-fluorphenyl)-3-(2-trifluormethylphenyl)-oxiran in 300 ml Methylenchlorid und 58 g Triethylamin werden bei Raumtemperatur 61 g 4-Methylbenzolsulfonsäurechlorid zugesetzt. Nach 24 Stunden wird das Reaktionsgemisch mit wässriger Natriumhydrogencarbonat-Lösung und Wasser gewaschen und im Vakuum eingedampft. Aus dem Rückstand erhielt man 128,4 g (95%) cis-2-(4-Methylphenylsulfonyloxymethyl)-2-(4-fluorphenyl)-3-(2-trifluormethylphenyl)-oxiran, das anschließend mit Triazol weiter verarbeitet wurde.

Beispiel D

Zu einer Lösung von 150 g E-2-(4-Fluorphenyl)-3-(2-chlorphenyl)-propenal (Darstellung analog Beispiel A) in 600 ml Isopropanol werden bei 0°C 7,3 g Natriumborhydrid gegeben, das in wenig 10 %iger Natronlauge gelöst war. Nachdem das Reaktionsgemisch zwei Stunden bei Raumtemperatur rührte, wird der Lösung 300 ml Wasser zugesetzt und die entstandene Emulsion mit Methylenchlorid ausgeschüttelt. Die isolierte organische Phase wird daraufhin über Natriumsulfat getrocknet und eingeengt. Man erhält 150,7 g (100 %) E-2-(4-Fluorphenyl)-3-(2-chlorphenyl)-prop-2-enol.

Beispiel E

103,1 g E-2-(4-Fluorphenyl)-3-(2-chlorphenyl)-prop-2-enol werden in 700 ml Methyl-tert.-butylether gelöst und 2 ml Pyridin zugesetzt. Die Reaktionslösung wird unter Stickstoffatmosphäre bei 0°C gerührt, während innerhalb von 90 Minuten 27,2 g Phosphortribromid zugegeben werden. Nach beendeter Zugabe wird zwei Stunden unter Rückfluß erhitzt, anschließend auf 500 ml Wasser gegeben und mehrmals mit Methyl-tert.-butylether extrahiert. Die organische Phase wird mit Natriumhydrogencarbonat-Lösung und Wasser gewaschen, über Natriumsulfat getrocknet und das Lösungsmittel im Vakuum eingedampft. Man erhält aus Ethanol 77,2 g (61 %) E-1-Brom-2-(4-fluorphenyl)-3-(2-chlorphenyl)-prop-2-en; Fp.: 68°C.

Beispiel F

77,2 g E-1-Brom-2-(4-fluorphenyl)-3-(2-chlorphenyl)-prop-2-en werden für eine Stunde auf 180°C erhitzt. Das Reaktionsgemisch wird auf Raumtemperatur gekühlt und mit 200 ml Ethanol versetzt. Anschließend werden 10 g Aktivkohle zugegeben, auf 50°C erhitzt und heiß filtriert. Man erhält aus Ethanol 26,9 g (35 %) Z-1-Brom-2-(4-fluorphenyl)-3-(2-chlorphenyl)prop-2-en mit dem Schmelzpunkt 73-75°C.

Beispiel G

78,2 g E-2-(4-Fluorphenyl)-3-(2-chlorphenyl)-propenal werden in 300 ml Methanol gelöst und 1 ml Natronlauge (konz.) zugesetzt. Die Reaktionslösung wird bei 0°C gerührt, während 20,5 g Wasserstoffperoxyd (ca. 50 %ig) langsam zugetropft werden, wobei die Innentemperatur von 30°C nicht überschritten wird. Nach beendeter Zugabe wird sechs Stunden bei Raumtemperatur gerührt, anschließend 100 ml Wasser zugesetzt und die entstandene Emulsion mit Methyl-tert.-butylether ausgeschüttelt. Die isolierte organische Phase wird daraufhin über Natriumsulfat getrocknet und eingeengt. Man erhält 52,5 g (63%) cis-2-Formyl-2-(4-fluorphenyl)-3-(2-chlorphenyl)-oxiran.

II. Herstellung der Endprodukte

Beispiel 1

55 g 4,5-Dichlorimidazol und 9,5 g Natriumhydrid (80 %ige Dispersion in Mineralöl) werden in 300 ml N,N-Dimethylformamid suspendiert und bei Raumtemperatur mit einer Lösung aus 128,4 g cis-2-(4-Methylphenylsulfonyloxy methyl)-2-(4-fluorphenyl)-3-(2-trifluormethylphenyl)-oxiran in 200 ml N,N-Dimethylformamid versetzt. Nach acht Stunden wird die Reaktionlösung auf Wasser gegeben und mit Methyl-tert.-butylether extrahiert. Die organische Phase wird mit Wasser gewaschen, über Natriumsulfat getrocknet und

das Lösungsmittel im Vakuum eingedampft. Man erhält aus Methyl-tert.-butylether/n-Hexan 86,4 g (76 %) cis-2-(4,5-Dichlorimidazol-1-yl-methyl)-2-(4-fluorphenyl)-3-(2-trifluormethylphenyl)-oxiran mit dem Schmelzpunkt 158°C (Verbindung Nr. 1).

Entsprechend Beispiel 1 können die in der Tabelle 1 aufgeführten Verbindungen hergestellt werden.

Beispiel 2

Eine Lösung von 7,5 g Z-1-Brom-2-(4-fluorphenyl)-3-(2-chlorphenyl)-prop-2-en in 40 ml Dimethylformamid wird mit 15 g 5-Nitro-imidazol und 31 g Kaliumcarbonat versetzt. Nachdem das Reaktionsgemisch 24 Stunden bei Raumtemperatur rührte, wird 50 ml Wasser zugesetzt und mehrmals mit Methyl-tert.-butylether extrahiert; die organische Phase mit Wasser gewaschen, über Natriumsulfat getrocknet und im Vakuum eingeengt. Man erhält aus Methyl-tert.-butylether/n-Hexan 6,7 g (81 %) Z-1-(5-Nitro-imidazol-1-yl)-2-(4-fluorphenyl)-3-(2-chlorphenyl)-prop-2-en mit dem Schmelzpunkt 161-165°C (Verbindung Nr. 1a).

Entsprechend Beispiel 2 können die in der Tabelle 2 aufgeführten Verbindungen hergestellt werden.

Beispiel 3

Zu einer Lösung von 27,7 g 4-Imidazolcarbonsäuremethylester in 150 ml Methylenchlorid wird bei 0°C 13,1 g Thionylchlorid zugesetzt. Nach beendeter Zugabe wird bei Raumtemperatur für 30 Minuten gerührt und anschließend 18,5 g cis-2-Formyl-2-(4-fluorphenyl)-3-(2-chlorphenyl)-oxiran zugegeben. Nachdem das Reaktionsgemisch 12-15 Stunden bei Raumtemperatur rührte, wird der Lösung 100 ml Wasser zugesetzt und die organische Phase abgetrennt. Die verbleibende wässrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet und eingeengt. Man erhält aus Methyl-tert.-butylether 14,3 g (53 %) cis-2-[1-(4-Methoxycarbonylimidazol-1-yl)-1-chlor-methyl]-2-(4-fluorphenyl)-3-(2-chlorphenyl)-oxiran mit dem Schmelzpunkt 199-203° C (Verbindung Nr. 25).

Entsprechend Beispiel 3 können die in der Tabelle 1 aufgeführten Verbindungen hergestellt werden.

Beispiel 4

Zu einer Lösung von 30,1 g 4,5-Dichlorimidazol in 150 ml Methylenchlorid wird bei 0°C 13,1 g Thionylchlorid zugesetzt. Nach beendeter Zugabe wird bei Raumtemperatur für 30 Minuten gerührt und anschließend 17,4 g E-2-(4-Fluorphenyl)-3-(2-chlorphenyl)-propenal zugegeben. Nachdem das Reaktionsgemisch 12-15 Stunden bei Raumtemperatur rührte, wird der Lösung 100 ml Wasser zugesetzt und die organische Phase abgetrennt. Die verbleibende wässrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet, eingeengt und durch Chromatographie an Kieselgel (Essigester/n-Hexan = 9:1) gereinigt. Man erhält 24,8 g (89 %) E-1-Chlor-1-(4,5-dichlorimidazol-1-yl)-2-(4-fluorphenyl)-3-(2-chlorphenyl)-prop-2-en (Verbindung Nr. 8a).

Endsprechend Beispiel 4 können die in der Tabelle 2 aufgeführten Verbindungen hergestellt werden.

Tabelle 1

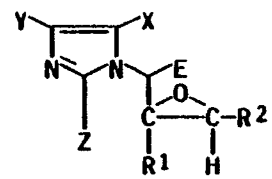

I

| Bsp. | $R^1$ | $R^2$ | E | X | Y | Z | Schmp/IR | Isomer |
|---|---|---|---|---|---|---|---|---|
| 1 | $4-F-C_6H_4$ | $2-CF_3-C_6H_4$ | H | Cl | Cl | H | 158°C | cis |
| 2 | $4-F-C_6H_4$ | $2-CF_3-C_6H_4$ | H | H | $CO_2CH_3$ | H | 1699, 1514, 1315, 1222, 1123, 771 $cm^{-1}$ | cis |
| 3 | $4-F-C_6H_4$ | $2-CF_3-C_6H_4$ | H | Cl | $CH_3$ | H | 164-167°C | cis |
| 4 | $4-F-C_6H_4$ | $2-CF_3-C_6H_4$ | H | H | H | $CH_3$ | | |
| 5 | $4-F-C_6H_4$ | $2-CF_3-C_6H_4$ | H | H | CN | H | | |
| 6 | $4-F-C_6H_4$ | $2-CF_3-C_6H_4$ | H | $NO_2$ | H | H | | |
| 7 | $4-F-C_6H_4$ | $2-CF_3-C_6H_4$ | H | H | $CH_3$ | H | | |
| 8 | $4-F-C_6H_4$ | $2-CF_3-C_6H_4$ | Cl | Cl | Cl | H | | |
| 9 | $4-F-C_6H_4$ | $2-CF_3-C_6H_4$ | Cl | H | $CO_2CH_3$ | H | | |
| 10 | $4-F-C_6H_4$ | $2-CF_3-C_6H_4$ | Cl | Cl | $CH_3$ | H | | |
| 11 | $4-F-C_6H_4$ | $2-CF_3-C_6H_4$ | Cl | H | H | $CH_3$ | | |
| 12 | $4-F-C_6H_4$ | $2-CF_3-C_6H_4$ | Cl | H | CN | H | | |
| 13 | $4-F-C_6H_4$ | $2-CF_3-C_6H_4$ | Cl | $NO_2$ | H | H | | |
| 14 | $4-F-C_6H_4$ | $2-CF_3-C_6H_4$ | Br | Cl | Cl | H | | |
| 15 | $4-F-C_6H_4$ | $2-CF_3-C_6H_4$ | Br | H | $CO_2CH_3$ | H | | |
| 16 | $4-F-C_6H_4$ | $2-CF_3-C_6H_4$ | Br | H | $CH_3$ | H | | |

Tabelle 1 - Forts.

| Bsp. | $R^1$ | $R^2$ | E | X | Y | Z | Schmp/IR | Isomer |
|---|---|---|---|---|---|---|---|---|
| 17 | $4-F-C_6H_4$ | $2-Cl-C_6H_4$ | H | Cl | Cl | H | 108-115°C | cis |
| 18 | $4-F-C_6H_4$ | $2-Cl-C_6H_4$ | H | H | $CO_2CH_3$ | H | Harz | cis |
| 19 | $4-F-C_6H_4$ | $2-Cl-C_6H_4$ | H | Cl | $CH_3$ | H | 112-113°C | cis |
| 20 | $4-F-C_6H_4$ | $2-Cl-C_6H_4$ | H | H | H | $CH_3$ | 100-103°C | cis |
| 21 | $4-F-C_6H_4$ | $2-Cl-C_6H_4$ | H | H | CN | H | 128-131°C | cis |
| 22 | $4-F-C_6H_4$ | $2-Cl-C_6H_4$ | H | H | $NO_2$ | H | 206-208°C | cis |
| 23 | $4-F-C_6H_4$ | $2-Cl-C_6H_4$ | H | H | $CH_3$ | H | Harz | cis |
| 24 | $4-F-C_6H_4$ | $2-Cl-C_6H_4$ | Cl | Cl | Cl | H | 156-158°C | cis |
| 25 | $4-F-C_6H_4$ | $2-Cl-C_6H_4$ | Cl | H | $CO_2CH_3$ | H | 199-203°C | cis |
| 26 | $4-F-C_6H_4$ | $2-Cl-C_6H_4$ | Cl | Cl | $CH_3$ | H | 146-156°C | D1:D2=1:1 |
| 27 | $4-F-C_6H_4$ | $2-Cl-C_6H_4$ | Cl | H | H | $CH_3$ | | |
| 28 | $4-F-C_6H_4$ | $2-Cl-C_6H_4$ | Cl | H | CN | H | 50-60°C | D1:D2=2:1 |
| 29 | $4-F-C_6H_4$ | $2-Cl-C_6H_4$ | Cl | $NO_2$ | H | H | | |
| 30 | $4-F-C_6H_4$ | $2-Cl-C_6H_4$ | Cl | H | $CH_3$ | H | | |
| 31 | $4-F-C_6H_4$ | $2-Cl-C_6H_4$ | Br | Cl | Cl | H | 115-122°C | D1:D2=4:1 |
| 32 | $4-F-C_6H_4$ | $2-Cl-C_6H_4$ | Br | H | $CO_2CH_3$ | H | 162°C | cis |
| 33 | $4-F-C_6H_4$ | $2-Cl-C_6H_4$ | Br | Cl | $CH_3$ | H | | |
| 34 | $4-F-C_6H_4$ | $2-Cl-C_6H_4$ | Br | H | H | $CH_3$ | | |
| 35 | $4-F-C_6H_4$ | $2-Cl-C_6H_4$ | Br | H | CN | H | | |
| 36 | $4-F-C_6H_4$ | $2-Cl-C_6H_4$ | Br | $NO_2$ | H | H | | |
| 37 | $4-F-C_6H_4$ | $4-Cl-C_6H_4$ | H | Cl | Cl | H | 146-148°C | cis |
| 38 | $4-F-C_6H_4$ | $4-Cl-C_6H_4$ | H | H | $CO_2CH_3$ | H | 138-141°C | cis |
| 39 | $4-F-C_6H_4$ | $4-Cl-C_6H_4$ | H | Cl | $CH_3$ | H | 124-126°C | cis |
| 40 | $4-F-C_6H_4$ | $4-Cl-C_6H_4$ | H | H | H | $CH_3$ | | |
| 41 | $4-F-C_6H_4$ | $4-Cl-C_6H_4$ | H | H | CN | H | | |

Tabelle 1 - Forts.

| Bsp. | $R^1$ | $R^2$ | E | X | Y | Z | Schmp/IR | Isomer |
|---|---|---|---|---|---|---|---|---|
| 42 | 4-F-$C_6H_4$ | 4-Cl-$C_6H_4$ | H | $NO_2$ | H | H | | |
| 43 | 4-F-$C_6H_4$ | 4-Cl-$C_6H_4$ | H | H | $COCH_3$ | H | 127-130°C | cis |
| 44 | 4-F-$C_6H_4$ | 4-Cl-$C_6H_4$ | Cl | Cl | Cl | H | | |
| 45 | 4-F-$C_6H_4$ | 4-Cl-$C_6H_4$ | Cl | H | $CO_2CH_3$ | H | | |
| 46 | 4-F-$C_6H_4$ | 4-Cl-$C_6H_4$ | Cl | Cl | $CH_3$ | H | | |
| 47 | 4-F-$C_6H_4$ | 4-Cl-$C_6H_4$ | Cl | H | H | $CH_3$ | | |
| 48 | 4-F-$C_6H_4$ | 4-Cl-$C_6H_4$ | Cl | H | CN | H | | |
| 49 | 4-F-$C_6H_4$ | 4-Cl-$C_6H_4$ | Cl | $NO_2$ | H | H | | |
| 50 | 4-F-$C_6H_4$ | 4-Cl-$C_6H_4$ | Br | Cl | Cl | H | | |
| 51 | 4-F-$C_6H_4$ | 4-Cl-$C_6H_4$ | Br | H | $CO_2CH_3$ | H | | |
| 52 | 4-F-$C_6H_4$ | 4-Cl-$C_6H_4$ | Br | Cl | $CH_3$ | H | | |
| 53 | 4-F-$C_6H_4$ | 4-Cl-$C_6H_4$ | Br | H | H | $CH_3$ | | |
| 54 | 4-F-$C_6H_4$ | 4-Cl-$C_6H_4$ | Br | H | CN | H | | |
| 55 | 4-F-$C_6H_4$ | 4-Cl-$C_6H_4$ | Br | $NO_2$ | H | H | | |
| 56 | 4-F-$C_6H_4$ | 4-Cl-$C_6H_4$ | Br | H | $CH_3$ | H | | |
| 57 | 4-F-$C_6H_4$ | 2-Cl-4-F-$C_6H_3$ | H | Cl | Cl | H | 105-110°C | cis |
| 58 | 4-F-$C_6H_4$ | 2-Cl-4-F-$C_6H_3$ | H | H | $CO_2CH_3$ | H | Harz | cis |
| 59 | 4-F-$C_6H_4$ | 2-Cl-4-F-$C_6H_3$ | H | Cl | $CH_3$ | H | 150°C | cis |
| 60 | 4-F-$C_6H_4$ | 2-Cl-4-F-$C_6H_3$ | H | H | H | $CH_3$ | | |
| 61 | 4-F-$C_6H_4$ | 2-Cl-4-F-$C_6H_3$ | H | H | CN | H | | |
| 62 | 4-F-$C_6H_4$ | 2-Cl-4-F-$C_6H_3$ | H | $NO_2$ | H | H | | |
| 63 | 4-F-$C_6H_4$ | 2-Cl-4-F-$C_6H_3$ | H | H | $CH_3$ | H | | |
| 64 | 4-F-$C_6H_4$ | 2-Cl-4-F-$C_6H_3$ | Cl | Cl | Cl | H | 145-149°C | D1:D2=2:1 |
| 65 | 4-F-$C_6H_4$ | 2-Cl-4-F-$C_6H_3$ | Cl | H | $CO_2CH_3$ | H | | |
| 66 | 4-F-$C_6H_4$ | 2-Cl-4-F-$C_6H_3$ | Cl | Cl | $CH_3$ | H | | |

Tabelle 1 - Forts.

| Bsp. | $R^1$ | $R^2$ | E | X | Y | Z | Schmp/IR | Isomer |
|---|---|---|---|---|---|---|---|---|
| 67 | 4-F-$C_6H_4$ | 2-Cl-4-F-$C_6H_3$ | Cl | H | H | $CH_3$ | | |
| 68 | 4-F-$C_6H_4$ | 2-Cl-4-F-$C_6H_3$ | Cl | H | CN | H | | |
| 69 | 4-F-$C_6H_4$ | 2-Cl-4-F-$C_6H_3$ | Cl | $NO_2$ | H | H | | |
| 70 | 4-F-$C_6H_4$ | 2-Cl-4-F-$C_6H_3$ | Cl | H | $CH_3$ | H | | |
| 71 | 4-F-$C_6H_4$ | 2-Cl-4-F-$C_6H_3$ | Br | Cl | Cl | H | | |
| 72 | 4-F-$C_6H_4$ | 2-Cl-4-F-$C_6H_3$ | Br | H | $CO_2CH_3$ | H | | |
| 73 | 4-F-$C_6H_4$ | 2-Cl-4-F-$C_6H_3$ | Br | Cl | $CH_3$ | H | | |
| 74 | 4-F-$C_6H_4$ | 2-Cl-4-F-$C_6H_3$ | Br | H | H | $CH_3$ | | |
| 75 | 4-F-$C_6H_4$ | 2-Cl-4-F-$C_6H_3$ | Br | H | CN | H | | |
| 76 | 4-F-$C_6H_4$ | 2-Cl-4-F-$C_6H_3$ | Br | $NO_2$ | H | H | | |
| 77 | 4-F-$C_6H_4$ | 2-Cl-4-F-$C_6H_3$ | Br | H | $CH_3$ | H | | |
| 78 | 4-F-$C_6H_4$ | 2-F-$C_6H_4$ | H | Cl | Cl | H | | |
| 79 | 4-F-$C_6H_4$ | 2-F-$C_6H_4$ | H | H | $CO_2CH_3$ | H | | |
| 80 | 4-F-$C_6H_4$ | 2-F-$C_6H_4$ | H | Cl | $CH_3$ | H | | |
| 81 | 4-F-$C_6H_4$ | 2-F-$C_6H_4$ | Cl | Cl | Cl | H | | |
| 82 | 4-F-$C_6H_4$ | 2-F-$C_6H_4$ | Cl | H | $CO_2CH_3$ | H | | |
| 83 | 4-F-$C_6H_4$ | 2-F-$C_6H_4$ | Cl | Cl | $CH_3$ | H | | |
| 84 | 4-F-$C_6H_4$ | 2-F-$C_6H_4$ | Br | Cl | Cl | H | | |
| 85 | 4-F-$C_6H_4$ | 2-F-$C_6H_4$ | Br | H | $CO_2CH_3$ | H | | |
| 86 | 4-F-$C_6H_4$ | 2-F-$C_6H_4$ | Br | H | $CH_3$ | H | | |
| 87 | 4-F-$C_6H_4$ | 4-F-$C_6H_4$ | H | Cl | Cl | H | | |
| 88 | 4-F-$C_6H_4$ | 4-F-$C_6H_4$ | H | H | $CO_2CH_3$ | H | | |
| 89 | 4-F-$C_6H_4$ | 4-F-$C_6H_4$ | H | Cl | $CH_3$ | H | | |
| 90 | 4-F-$C_6H_4$ | 4-F-$C_6H_4$ | H | H | $CH_3$ | H | | |

Tabelle 1 - Forts.

| Bsp. | $R^1$ | $R^2$ | E | X | Y | Z | Schmp/IR | Isomer |
|---|---|---|---|---|---|---|---|---|
| 91 | 4-F-$C_6H_4$ | 4-F-$C_6H_4$ | Cl | Cl | Cl | H | | |
| 92 | 4-F-$C_6H_4$ | 4-F-$C_6H_4$ | Cl | H | $CO_2CH_3$ | H | | |
| 93 | 4-F-$C_6H_4$ | 4-F-$C_6H_4$ | Cl | Cl | $CH_3$ | H | | |
| 94 | 4-F-$C_6H_4$ | 4-F-$C_6H_4$ | Cl | H | $CH_3$ | H | | |
| 95 | 4-F-$C_6H_4$ | 4-F-$C_6H_4$ | Br | Cl | Cl | H | | |
| 96 | 4-F-$C_6H_4$ | 4-F-$C_6H_4$ | Br | H | $CO_2CH_3$ | H | | |
| 97 | 4-F-$C_6H_4$ | 4-F-$C_6H_4$ | Br | Cl | $CH_3$ | H | | |
| 98 | 4-F-$C_6H_4$ | 4-F-$C_6H_4$ | Br | H | $CH_3$ | H | | |
| 99 | 4-F-$C_6H_4$ | 2,4-$F_2$-$C_6H_3$ | H | Cl | Cl | H | Harz | cis |
| 100 | 4-F-$C_6H_4$ | 2,4-$F_2$-$C_6H_3$ | H | H | $CO_2CH_3$ | H | 141-144°C | cis |
| 101 | 4-F-$C_6H_4$ | 2,4-$F_2$-$C_6H_3$ | H | Cl | $CH_3$ | H | 144-147°C | cis |
| 102 | 4-F-$C_6H_4$ | 2,4-$F_2$-$C_6H_3$ | H | H | $COCH_3$ | H | Harz | cis |
| 103 | 4-F-$C_6H_4$ | 2,4-$F_2$-$C_6H_3$ | H | H | H | $CH_3$ | | |
| 104 | 4-F-$C_6H_4$ | 2,4-$F_2$-$C_6H_3$ | Cl | Cl | Cl | H | 1607, 1507, 1463, 1237, 964 $cm^{-1}$ | $D_1$:$D_2$=2:1 |
| 105 | 4-F-$C_6H_4$ | 2,4-$F_2$-$C_6H_3$ | Cl | H | $CO_2CH_3$ | H | | |
| 106 | 4-F-$C_6H_4$ | 2,4-$F_2$-$C_6H_3$ | Cl | Cl | $CH_3$ | H | | |
| 107 | 4-F-$C_6H_4$ | 2,4-$F_2$-$C_6H_3$ | Cl | H | $CH_3$ | H | | |
| 108 | 4-F-$C_6H_4$ | 2,4-$F_2$-$C_6H_3$ | Cl | H | H | $CH_3$ | | |
| 109 | 4-F-$C_6H_4$ | 2,4-$F_2$-$C_6H_3$ | Br | Cl | Cl | H | | |
| 110 | 4-F-$C_6H_4$ | 2,4-$F_2$-$C_6H_3$ | Br | H | $CO_2CH_3$ | H | | |
| 111 | 4-F-$C_6H_4$ | 2,4-$F_2$-$C_6H_3$ | Br | Cl | $CH_3$ | H | | |
| 112 | 4-F-$C_6H_4$ | 2,6-$F_2$-$C_6H_3$ | H | Cl | Cl | H | 131-132°C | cis |
| 113 | 4-F-$C_6H_4$ | 2,6-$F_2$-$C_6H_3$ | H | H | $CO_2CH_3$ | H | Harz | cis |
| 114 | 4-F-$C_6H_4$ | 2,6-$F_2$-$C_6H_3$ | H | Cl | $CH_3$ | H | 129-132°C | cis |

Tabelle 1 - Forts.

| Bsp. | R1 | R2 | E | X | Y | Z | Schmp/IR | Isomer |
|---|---|---|---|---|---|---|---|---|
| 115 | $4-F-C_6H_4$ | $2,6-F_2-C_6H_3$ | H | H | $COCH_3$ | H | Harz | cis |
| 116 | $4-F-C_6H_4$ | $2,6-F_2-C_6H_3$ | Cl | H | $CO_2CH_3$ | H | | |
| 117 | $4-F-C_6H_4$ | $2,6-F_2-C_6H_3$ | Cl | Cl | $CH_3$ | H | | |
| 118 | $4-F-C_6H_4$ | $2,6-F_2-C_6H_3$ | Br | Cl | Cl | H | | |
| 119 | $4-F-C_6H_4$ | $2,6-F_2-C_6H_3$ | Br | H | $CO_2CH_3$ | H | | |
| 120 | $4-F-C_6H_4$ | $2,6-F_2-C_6H_3$ | Br | H | H | $CH_3$ | | |
| 121 | $4-F-C_6H_4$ | $2,4-Cl_2-C_6H_3$ | H | Cl | Cl | H | | |
| 122 | $4-F-C_6H_4$ | $2,4-Cl_2-C_6H_3$ | H | H | $CO_2CH_3$ | H | | |
| 123 | $4-F-C_6H_4$ | $2,4-Cl_2-C_6H_3$ | H | Cl | $CH_3$ | H | | |
| 124 | $4-F-C_6H_4$ | $2,4-Cl_2-C_6H_3$ | Cl | Cl | Cl | H | | |
| 125 | $4-F-C_6H_4$ | $2,4-Cl_2-C_6H_3$ | Cl | H | $CO_2CH_3$ | H | | |
| 126 | $4-F-C_6H_4$ | $2,4-Cl_2-C_6H_3$ | Cl | Cl | $CH_3$ | H | | |
| 127 | $4-F-C_6H_4$ | $2,4-Cl_2-C_6H_3$ | Br | Cl | Cl | H | | |
| 128 | $4-F-C_6H_4$ | $2,4-Cl_2-C_6H_3$ | Br | H | $CO_2CH_3$ | H | | |
| 129 | $4-F-C_6H_4$ | $2,4-Cl_2-C_6H_3$ | Br | Cl | $CH_3$ | H | | |
| 130 | $4-F-C_6H_4$ | $2-OCH_3-C_6H_4$ | H | Cl | Cl | H | | |
| 131 | $4-F-C_6H_4$ | $2-OCH_3-C_6H_4$ | H | H | $CO_2CH_3$ | H | 1731, 1514, 1494, 1245, 1223, 758 $cm^{-1}$ | cis |
| 132 | $4-F-C_6H_4$ | $2-OCH_3-C_6H_4$ | H | Cl | $CH_3$ | H | | |
| 133 | $4-F-C_6H_4$ | $2-OCH_3-C_6H_4$ | H | H | $CH_3$ | H | | |
| 134 | $4-F-C_6H_4$ | $2-OCH_3-C_6H_4$ | H | H | H | $CH_3$ | | |
| 135 | $4-F-C_6H_4$ | $2-OCH_3-C_6H_4$ | H | H | CN | H | | |
| 136 | $4-F-C_6H_4$ | $2-OCH_3-C_6H_4$ | H | $NO_2$ | H | H | | |
| 137 | $4-F-C_6H_4$ | $2-OCH_3-C_6H_4$ | Cl | Cl | Cl | H | 146-149°C | cis |
| 138 | $4-F-C_6H_4$ | $2-OCH_3-C_6H_4$ | Cl | H | $CO_2CH_3$ | H | | |

Tabelle 1 - Forts.

| Bsp. | $R^1$ | $R^2$ | E | X | Y | Z | Schmp/IR | Isomer |
|---|---|---|---|---|---|---|---|---|
| 139 | $4-F-C_6H_4$ | $2-OCH_3-C_6H_4$ | Cl | Cl | $CH_3$ | H | | |
| 140 | $4-F-C_6H_4$ | $2-OCH_3-C_6H_4$ | Cl | H | $CH_3$ | H | | |
| 141 | $4-F-C_6H_4$ | $2-OCH_3-C_6H_4$ | Cl | H | H | $CH_3$ | | |
| 142 | $4-F-C_6H_4$ | $2-OCH_3-C_6H_4$ | Cl | H | CN | H | | |
| 143 | $4-F-C_6H_4$ | $2-OCH_3-C_6H_4$ | Cl | $NO_2$ | H | H | | |
| 144 | $4-F-C_6H_4$ | $2-OCH_3-C_6H_4$ | Br | Cl | Cl | H | Harz | D1:D2=3:1 |
| 145 | $4-F-C_6H_4$ | $2-OCH_3-C_6H_4$ | Br | H | $CO_2CH_3$ | H | | |
| 146 | $4-F-C_6H_4$ | $2-OCH_3-C_6H_4$ | Br | Cl | $CH_3$ | H | | |
| 147 | $4-F-C_6H_4$ | $4-OCF_3-C_6H_4$ | H | Cl | Cl | H | | |
| 148 | $4-F-C_6H_4$ | $4-OCF_3-C_6H_4$ | H | H | $CO_2CH_3$ | H | | |
| 149 | $4-F-C_6H_4$ | $4-OCF_3-C_6H_4$ | H | Cl | $CH_3$ | H | | |
| 150 | $4-F-C_6H_4$ | $4-OCF_3-C_6H_4$ | H | H | $CH_3$ | H | | |
| 151 | $4-F-C_6H_4$ | $4-OCF_3-C_6H_4$ | H | H | H | $CH_3$ | | |
| 152 | $4-F-C_6H_4$ | $4-OCF_3-C_6H_4$ | H | H | CN | H | | |
| 153 | $4-F-C_6H_4$ | $4-OCF_3-C_6H_4$ | Cl | Cl | Cl | H | | |
| 154 | $4-F-C_6H_4$ | $4-OCF_3-C_6H_4$ | Cl | H | $CO_2CH_3$ | H | | |
| 155 | $4-F-C_6H_4$ | $4-OCF_3-C_6H_4$ | Cl | Cl | $CH_3$ | H | | |
| 156 | $4-F-C_6H_4$ | $4-OCF_3-C_6H_4$ | Cl | H | $CH_3$ | H | | |
| 157 | $4-F-C_6H_4$ | $4-OCF_3-C_6H_4$ | Br | Cl | Cl | H | | |
| 158 | $4-F-C_6H_4$ | $4-OCF_3-C_6H_4$ | Br | H | $CO_2CH_3$ | H | | |
| 159 | $4-F-C_6H_4$ | $4-OCF_3-C_6H_4$ | Br | Cl | $CH_3$ | H | | |
| 160 | $4-F-C_6H_4$ | $4-OCF_3-C_6H_4$ | Br | H | $CH_3$ | H | | |
| 161 | $4-F-C_6H_4$ | $C_6H_5$ | H | Cl | Cl | H | 125-128°C | cis |
| 162 | $4-F-C_6H_4$ | $C_6H_5$ | H | H | $CO_2CH_3$ | H | 116-120°C | cis |

Tabelle 1 - Forts.

| Bsp. | $R^1$ | $R^2$ | E | X | Y | Z | Schmp/IR | Isomer |
|---|---|---|---|---|---|---|---|---|
| 163 | 4-F-$C_6H_4$ | $C_6H_5$ | H | Cl | $CH_3$ | H | | |
| 164 | 4-F-$C_6H_4$ | $C_6H_5$ | H | H | $CH_3$ | H | | |
| 165 | 4-F-$C_6H_4$ | $C_6H_5$ | Cl | Cl | Cl | H | | |
| 166 | 4-F-$C_6H_4$ | $C_6H_5$ | Cl | H | $CO_2CH_3$ | H | | |
| 167 | 4-F-$C_6H_4$ | $C_6H_5$ | Cl | Cl | $CH_3$ | H | | |
| 168 | 4-F-$C_6H_4$ | $C_6H_5$ | Cl | H | $CH_3$ | H | | |
| 169 | 4-F-$C_6H_4$ | $C_6H_5$ | Br | Cl | Cl | H | | |
| 170 | 4-F-$C_6H_4$ | $C_6H_5$ | Br | H | $CO_2CH_3$ | H | | |
| 171 | 4-F-$C_6H_4$ | $C_6H_5$ | Br | Cl | $CH_3$ | H | | |
| 172 | 4-F-$C_6H_4$ | $C_6H_5$ | Br | H | $CH_3$ | H | | |
| 173 | 4-F-$C_6H_4$ | Cyclohexyl | H | Cl | Cl | H | 2929,1513,1253, 837 $cm^{-1}$ | cis |
| 174 | 4-F-$C_6H_4$ | Cyclohexyl | H | H | $CO_2CH_3$ | H | | |
| 175 | 4-F-$C_6H_4$ | Cyclohexyl | H | Cl | $CH_3$ | H | | |
| 176 | 4-F-$C_6H_4$ | Cyclohexyl | Cl | Cl | Cl | H | | |
| 177 | 4-F-$C_6H_4$ | Cyclohexyl | Cl | H | $CO_2CH_3$ | H | | |
| 178 | 4-F-$C_6H_4$ | Cyclohexyl | Cl | Cl | $CH_3$ | H | | |
| 179 | 4-F-$C_6H_4$ | Cyclohexyl | Br | Cl | Cl | H | | |
| 180 | 4-F-$C_6H_4$ | Cyclohexyl | Br | H | $CO_2CH_3$ | H | | |
| 181 | $C_6H_5$ | 2-Cl-$C_6H_4$ | H | Cl | Cl | H | 124-128°C | cis |
| 182 | $C_6H_5$ | 2-Cl-$C_6H_4$ | H | H | $CO_2CH_3$ | H | Harz | cis |
| 183 | $C_6H_5$ | 2-Cl-$C_6H_4$ | H | Cl | $CH_3$ | H | 142°C | cis |
| 184 | $C_6H_5$ | 2-Cl-$C_6H_4$ | H | H | H | $CH_3$ | 89-90°C | cis |
| 185 | $C_6H_5$ | 2-Cl-$C_6H_4$ | H | H | CN | H | 105-110°C | cis |

Tabelle 1 - Forts.

| Bsp. | $R^1$ | $R^2$ | E | X | Y | Z | Schmp/IR | Isomer |
|---|---|---|---|---|---|---|---|---|
| 186 | $C_6H_5$ | $2-Cl-C_6H_4$ | H | $NO_2$ | H | H | 200°C | cis |
| 187 | $C_6H_5$ | $2-Cl-C_6H_4$ | H | H | $CH_3$ | H | 91-95°C | cis/trans= 1,5:1 |
| 188 | $C_6H_5$ | $2-Cl-C_6H_4$ | Cl | Cl | Cl | H | | |
| 189 | $C_6H_5$ | $2-Cl-C_6H_4$ | Cl | H | $CO_2CH_3$ | H | | |
| 190 | $C_6H_5$ | $2-Cl-C_6H_4$ | Cl | Cl | $CH_3$ | H | | |
| 191 | $C_6H_5$ | $2-Cl-C_6H_4$ | Br | Cl | Cl | H | | |
| 192 | $C_6H_5$ | $2-Cl-C_6H_4$ | Br | H | $CO_2CH_3$ | H | | |
| 193 | $C_6H_5$ | $C_6H_5$ | H | Cl | Cl | H | | |
| 194 | $C_6H_5$ | $C_6H_5$ | H | H | $CO_2CH_3$ | H | | |
| 195 | $C_6H_5$ | $C_6H_5$ | Cl | Cl | Cl | H | | |
| 196 | $C_6H_5$ | $C_6H_5$ | Cl | H | $CO_2CH_3$ | H | | |
| 197 | $C_6H_5$ | $C_6H_5$ | Br | Cl | Cl | H | | |
| 198 | $C_6H_5$ | $C_6H_5$ | Br | H | $CO_2CH_3$ | H | | |
| 199 | $C_6H_5$ | $4-Cl-C_6H_4$ | H | Cl | Cl | H | | |
| 200 | $C_6H_5$ | $4-Cl-C_6H_4$ | Cl | Cl | Cl | H | | |
| 201 | $C_6H_5$ | $4-Cl-C_6H_4$ | Br | Cl | Cl | H | | |
| 202 | $C_6H_5$ | $2,4-Cl_2-C_6H_3$ | H | H | $CO_2CH_3$ | H | | |
| 203 | $C_6H_5$ | $2,4-Cl_2-C_6H_3$ | Cl | Cl | Cl | H | | |
| 204 | $C_6H_5$ | $2,4-Cl_2-C_6H_3$ | Br | Cl | $CH_3$ | H | | |
| 205 | $C_6H_5$ | $4-NO_2-C_6H_4$ | H | Cl | Cl | H | | |
| 206 | $C_6H_5$ | $3-NO_2-C_6H_4$ | H | H | $CH_3$ | H | | |
| 207 | $C_6H_5$ | Cyclohexyl | H | $CH_3$ | Cl | H | | |
| 208 | $4-Cl-C_6H_4$ | $C_6H_5$ | H | Cl | Cl | H | | |

Tabelle 1 - Forts.

| Bsp. | $R^1$ | $R^2$ | E | X | Y | Z | Schmp/IR | Isomer |
|---|---|---|---|---|---|---|---|---|
| 209 | 4-Cl-$C_6H_4$ | $C_6H_5$ | Cl | Cl | Cl | H | | |
| 210 | 4-Cl-$C_6H_4$ | $C_6H_5$ | Br | H | $CH_3$ | H | | |
| 211 | 4-Cl-$C_6H_4$ | 4-Cl-$C_6H_4$ | H | Cl | Cl | H | | |
| 212 | 4-Cl-$C_6H_4$ | 4-Cl-$C_6H_4$ | Cl | Cl | Cl | H | | |
| 213 | 4-Cl-$C_6H_4$ | 4-Cl-$C_6H_4$ | Br | H | H | $CH_3$ | | |
| 214 | 4-Cl-$C_6H_4$ | 2,4-$Cl_2$-$C_6H_3$ | H | Cl | Cl | H | | |
| 215 | 4-Cl-$C_6H_4$ | 2,4-$Cl_2$-$C_6H_3$ | Cl | Cl | Cl | H | | |
| 216 | 4-Cl-$C_6H_4$ | 4-F-$C_6H_4$ | Cl | Cl | Cl | H | | |
| 217 | 4-Cl-$C_6H_4$ | 4-F-$C_6H_4$ | H | Cl | Cl | H | | |
| 218 | 4-Cl-$C_6H_4$ | 4-F-$C_6H_4$ | Br | H | $CO_2CH_3$ | H | | |
| 219 | 4-Cl-$C_6H_4$ | 2-F-$C_6H_4$ | H | Cl | Cl | H | | |
| 220 | 4-Cl-$C_6H_4$ | 2-F-$C_6H_4$ | Cl | Cl | Cl | H | | |
| 221 | 2-Cl-$C_6H_4$ | 2-Cl-$C_6H_4$ | H | Cl | Cl | H | | |
| 222 | 2-Cl-$C_6H_4$ | 2-Cl-$C_6H_4$ | Cl | Cl | Cl | H | | |
| 223 | 2-Cl-$C_6H_4$ | 2-Cl-$C_6H_4$ | Br | H | $CH_3$ | H | | |
| 224 | 2-Cl-$C_6H_4$ | 2-F-$C_6H_4$ | H | Cl | Cl | H | | |
| 225 | 2-Cl-$C_6H_4$ | 2-F-$C_6H_4$ | Cl | Cl | Cl | H | | |
| 226 | 2-Cl-$C_6H_4$ | 4-Cl-$C_6H_4$ | H | Cl | Cl | H | | |
| 227 | 2-Cl-$C_6H_4$ | 4-Cl-$C_6H_4$ | Cl | Cl | Cl | H | | |
| 228 | 2-F-$C_6H_4$ | 2-Cl-$C_6H_4$ | H | Cl | Cl | H | 87-94°C | cis:trans=3:1 |
| 229 | 2-F-$C_6H_4$ | 2-Cl-$C_6H_4$ | Cl | H | $CH_3$ | H | | |
| 230 | 2-F-$C_6H_4$ | 4-F-$C_6H_4$ | H | Cl | Cl | H | | |
| 231 | 2-F-$C_6H_4$ | 4-F-$C_6H_4$ | Cl | Cl | Cl | H | | |
| 232 | 2-F-$C_6H_4$ | 4-Cl-$C_6H_4$ | H | Cl | Cl | H | | |

Tabelle 1 - Forts.

| Bsp. | $R^1$ | $R^2$ | E | X | Y | Z | Schmp/IR | Isomer |
|---|---|---|---|---|---|---|---|---|
| 233 | 2-F-$C_6H_4$ | 4-Cl-$C_6H_4$ | Cl | Cl | Cl | H | | |
| 234 | 2,4-$Cl_2$-$C_6H_3$ | 2-Cl-$C_6H_4$ | H | H | $CH_3$ | H | | |
| 235 | 2,4-$Cl_2$-$C_6H_3$ | 2-Cl-$C_6H_4$ | Cl | Cl | Cl | H | | |
| 236 | 2,4-$Cl_2$-$C_6H_3$ | 4-Cl-$C_6H_4$ | H | H | H | $CH_3$ | | |
| 237 | 2,4-$Cl_2$-$C_6H_3$ | 4-Cl-$C_6H_4$ | Cl | Cl | Cl | H | | |
| 238 | 2,4-$Cl_2$-$C_6H_3$ | 2,4-$Cl_2$-$C_6H_3$ | H | Cl | Cl | H | | |
| 239 | 2,4-$Cl_2$-$C_6H_3$ | 2,4-$Cl_2$-$C_6H_3$ | Cl | Cl | Cl | H | | |
| 240 | 2,4-$Cl_2$-$C_6H_3$ | 4-F-$C_6H_4$ | H | H | $CO_2CH_3$ | H | | |
| 241 | 2,4-$Cl_2$-$C_6H_3$ | 4-F-$C_6H_4$ | Br | Cl | $CH_3$ | H | | |
| 242 | Cyclohexyl | $C_6H_5$ | H | Cl | Cl | H | | |
| 243 | Cyclohexyl | $C_6H_5$ | Cl | H | CN | H | | |
| 244 | Cyclohexyl | $C_6H_5$ | Br | H | H | $CH_3$ | | |
| 245 | Cyclohexyl | 2-Cl-$C_6H_4$ | H | Cl | Cl | H | | |
| 246 | Cyclohexyl | 2-Cl-$C_6H_4$ | Cl | Cl | Cl | H | | |
| 247 | Cyclohexyl | 2-Cl-$C_6H_4$ | Br | H | $CH_3$ | H | | |
| 248 | Cyclohexyl | 4-Cl-$C_6H_4$ | H | Cl | Cl | H | | |
| 249 | Cyclohexyl | 4-Cl-$C_6H_4$ | Cl | Cl | Cl | H | | |
| 250 | Cyclohexyl | 2,4-$Cl_2$-$C_6H_3$ | H | H | $CO_2CH_3$ | H | | |
| 251 | Cyclohexyl | 2,4-$Cl_2$-$C_6H_3$ | Cl | H | $CO_2CH_3$ | H | | |
| 252 | Cyclohexyl | 2-F-$C_6H_4$ | H | H | $CH_3$ | H | | |
| 253 | Cyclohexyl | 2-F-$C_6H_4$ | Cl | $NO_2$ | H | H | | |
| 254 | Cyclohexyl | 4-F-$C_6H_4$ | H | Cl | Cl | H | | |
| 255 | Cyclohexyl | 4-F-$C_6H_4$ | Cl | Cl | Cl | H | | |
| 256 | Cyclohexyl | 4-F-$C_6H_4$ | Br | Cl | Cl | H | | |

Tabelle 1 - Forts.

| Bsp. | $R^1$ | $R^2$ | E | X | Y | Z | Schmp/IR | Isomer |
|---|---|---|---|---|---|---|---|---|
| 257 | Cyclohexyl | 2-Cl-4-F-$C_6H_3$ | H | H | $CO_2CH_3$ | H | | |
| 258 | Cyclohexyl | 2-Cl-4-F-$C_6H_3$ | Cl | Cl | Cl | H | | |
| 259 | Cyclohexyl | 2-Cl-4-F-$C_6H_3$ | Br | Cl | $CH_3$ | H | | |
| 260 | tert.-$C_4H_9$ | $C_6H_5$ | H | H | H | $CH_3$ | | |
| 261 | tert.-$C_4H_9$ | $C_6H_5$ | Cl | Cl | Cl | H | | |
| 262 | tert.-$C_4H_9$ | 2-Cl-$C_6H_4$ | H | Cl | Cl | H | | |
| 263 | tert.-$C_4H_9$ | 2-Cl-$C_6H_4$ | Cl | Cl | $CH_3$ | H | | |
| 264 | tert.-$C_4H_9$ | 4-Cl-$C_6H_4$ | H | Cl | Cl | H | | |
| 265 | tert.-$C_4H_9$ | 4-Cl-$C_6H_4$ | H | H | $CO_2CH_3$ | H | | |
| 266 | tert.-$C_4H_9$ | 4-Cl-$C_6H_4$ | H | Cl | $CH_3$ | H | | |
| 267 | tert.-$C_4H_9$ | 4-Cl-$C_6H_4$ | H | H | $CH_3$ | H | | |
| 268 | tert.-$C_4H_9$ | 4-Cl-$C_6H_4$ | H | H | H | $CH_3$ | | |
| 269 | tert.-$C_4H_9$ | 4-Cl-$C_6H_4$ | Cl | Cl | Cl | H | | |
| 270 | tert.-$C_4H_9$ | 4-Cl-$C_6H_4$ | Cl | H | $CO_2CH_3$ | H | | |
| 271 | tert.-$C_4H_9$ | 4-Cl-$C_6H_4$ | Cl | Cl | $CH_3$ | H | | |
| 272 | tert.-$C_4H_9$ | 4-Cl-$C_6H_4$ | Cl | H | $CH_3$ | H | | |
| 273 | tert.-$C_4H_9$ | 4-Cl-$C_6H_4$ | Cl | H | H | $CH_3$ | | |
| 274 | tert.-$C_4H_9$ | 4-Cl-$C_6H_4$ | Br | Cl | Cl | H | | |
| 275 | tert.-$C_4H_9$ | 4-Cl-$C_6H_4$ | Br | H | $CO_2CH_3$ | H | | |
| 276 | tert.-$C_4H_9$ | 4-Cl-$C_6H_4$ | Br | Cl | $CH_3$ | H | | |
| 277 | 2,4-$Cl_2$-$C_6H_3$ | 2-Cl-$C_6H_4$ | H | Cl | Cl | H | | |
| 278 | 2,4-$Cl_2$-$C_6H_3$ | 2-Cl-$C_6H_4$ | Cl | H | $CO_2CH_3$ | H | | |
| 279 | 2,4-$Cl_2$-$C_6H_3$ | 4-Cl-$C_6H_4$ | H | Cl | Cl | H | | |
| 280 | 2,4-$Cl_2$-$C_6H_3$ | 4-Cl-$C_6H_4$ | Cl | Cl | Cl | H | | |

Tabelle 1 - Forts.

| Bsp. | $R^1$ | $R^2$ | E | X | Y | Z | Schmp/IR | Isomer |
|---|---|---|---|---|---|---|---|---|
| 281 | $2,4-Cl_2-C_6H_3$ | $2,4-Cl_2-C_6H_3$ | Cl | Cl | Cl | H | | |
| 282 | $2,4-Cl_2-C_6H_3$ | $2,4-Cl_2-C_6H_3$ | H | H | $CH_3$ | H | | |
| 283 | $2,4-Cl_2-C_6H_3$ | $4-F-C_6H_4$ | H | Cl | $CH_3$ | H | | |
| 284 | $2,4-Cl_2-C_6H_3$ | $2-F-C_6H_4$ | H | H | H | $CH_3$ | | |
| 285 | $4-Br-C_6H_4$ | $4-C(CH_3)_3-C_6H_4$ | H | H | $CH_3$ | H | | |
| 286 | $2-OCH_3-C_6H_4$ | $2-Cl-C_6H_4$ | H | Cl | Cl | H | | |
| 287 | $2-OCH_3-C_6H_4$ | $4-Cl-C_6H_4$ | Cl | H | $CO_2CH_3$ | H | | |
| 288 | $2-OCH_3-C_6H_4$ | $2-C_{10}H_7$ | H | Cl | Cl | H | | |
| 289 | $2-OCH_3-C_6H_4$ | $4-F-C_6H_4$ | H | H | $CO_2CH_3$ | H | | |
| 290 | $2-OCH_3-C_6H_4$ | $2-F-C_6H_4$ | H | Cl | Cl | H | | |
| 291 | $2-C_{10}H_7$ | $2-Cl-C_6H_4$ | H | Cl | Cl | H | | |
| 292 | $2-C_{10}H_7$ | $4-F-C_6H_4$ | Cl | Cl | Cl | H | | |
| 293 | $4-F-C_6H_4$ | $2-Cl-C_6H_4$ | H | H | $COCH_3$ | $CH_3$ | 130-131°C | cis |
| 294 | $4-F-C_6H_4$ | $2-Cl-C_6H_4$ | Cl | H | $COCH_3$ | $CH_3$ | | |
| 295 | $4-F-C_6H_4$ | $2-Cl-C_6H_4$ | Br | H | $COCH_3$ | $CH_3$ | | |
| 296 | $4-F-C_6H_4$ | $2-CF_3-C_6H_4$ | F | Cl | Cl | H | | |
| 297 | $4-F-C_6H_4$ | $2-Cl-C_6H_4$ | F | Cl | $CH_3$ | H | | |
| 298 | $4-F-C_6H_4$ | $2-Cl-C_6H_4$ | F | Cl | Cl | H | | |
| 299 | $4-F-C_6H_4$ | $2-Cl-C_6H_4$ | F | H | CN | H | | |
| 300 | $4-F-C_6H_4$ | $4-Cl-C_6H_4$ | F | Cl | Cl | H | | |
| 301 | $4-F-C_6H_4$ | $4-Cl-C_6H_4$ | F | Cl | $CH_3$ | H | | |
| 302 | $4-F-C_6H_4$ | $2-Cl-4-F-C_6H_3$ | F | Cl | Cl | H | | |
| 303 | $4-F-C_6H_4$ | $2-F-C_6H_4$ | F | H | CN | H | | |
| 304 | $4-F-C_6H_4$ | $4-F-C_6H_4$ | F | Cl | $CH_3$ | H | | |

Tabelle 1 - Forts.

| Bsp. | $R^1$ | $R^2$ | E | X | Y | Z | Schmp/IR | Isomer |
|---|---|---|---|---|---|---|---|---|
| 305 | 4-F-$C_6H_4$ | 2,4-$F_2$-$C_6H_3$ | F | Cl | Cl | H | | |
| 306 | 4-F-$C_6H_4$ | 2,6-$F_2$-$C_6H_3$ | F | Cl | Cl | H | | |
| 307 | 4-F-$C_6H_4$ | 2,4-$Cl_2$-$C_6H_3$ | F | H | $COCH_3$ | $CH_3$ | | |
| 308 | 4-F-$C_6H_4$ | 2-$OCH_3$-$C_6H_4$ | F | Cl | Cl | H | | |
| 309 | 4-F-$C_6H_4$ | 4-$OCH_3$-$C_6H_4$ | F | H | CN | H | | |
| 310 | 4-F-$C_6H_4$ | $C_6H_5$ | F | Cl | $CH_3$ | H | | |
| 311 | 4-F-$C_6H_4$ | Cyclohexyl | F | Cl | Cl | H | | |
| 312 | $C_6H_5$ | 4-Cl-$C_6H_4$ | F | Cl | Cl | H | | |
| 313 | 4-Cl-$C_6H_4$ | 4-Cl-$C_6H_4$ | F | Cl | $CH_3$ | H | | |
| 314 | Cyclohexyl | 4-F-$C_6H_4$ | F | Cl | Cl | H | | |
| 315 | 4-F-$C_6H_4$ | 2-Cl-$C_6H_4$ | H | H | $COCH_3$ | H | Harz | cis |
| 316 | 4-F-$C_6H_4$ | 2-Cl-$C_6H_4$ | H | $COCH_3$ | $CH_3$ | H | 136-141°C | cis |
| 317 | 4-F-$C_6H_4$ | 2-Cl-$C_6H_4$ | H | H | Phenyl | H | Harz | D1:D2=2:1 |
| 318 | 4-F-$C_6H_4$ | 2-Cl-$C_6H_4$ | H | H | H | Phenyl | | |
| 319 | 4-F-$C_6H_4$ | $C_6H_5$ | H | H | CN | H | 126-130°C | cis |
| 320 | 4-F-$C_6H_4$ | 2-F-3Cl-$C_6H_3$ | H | Cl | Cl | H | 150-153°C | cis |
| 321 | 4-F-$C_6H_4$ | 4-$OCF_3$-$C_6H_4$ | H | H | $CO_2CH_3$ | H | Harz | cis |
| 322 | $C_6H_5$ | 2-$OCH_3$-$C_6H_4$ | H | Cl | Cl | H | Harz | cis |
| 323 | 4-F-$C_6H_4$ | 2-Br-$C_6H_4$ | H | H | $CO_2CH_3$ | H | Harz | cis |
| 324 | 4-F-$C_6H_4$ | 2-Cl-$C_6H_4$ | H | $CO_2CH_3$ | $CO_2CH_3$ | H | 134-140°C | cis |

Tabelle 2

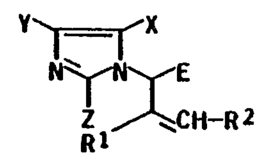

| Bsp. | $R^1$ | $R^2$ | E | X | Y | Z | Schmp/IR | Isomer |
|---|---|---|---|---|---|---|---|---|
| 1a | 4-F-$C_6H_4$ | 2-Cl-$C_6H_4$ | H | $NO_2$ | H | H | 161-165°C | Z |
| 2a | 4-F-$C_6H_4$ | 2-Cl-$C_6H_4$ | H | H | $CO_2CH_3$ | H | 132-135°C | Z |
| 3a | 4-F-$C_6H_4$ | 2-Cl-$C_6H_4$ | H | Cl | Cl | H | 84- 89°C | Z |
| 4a | 4-F-$C_6H_4$ | 2-Cl-$C_6H_4$ | H | H | CN | H | 99-108°C | Z/E=6:1 |
| 5a | 4-F-$C_6H_4$ | 2-Cl-$C_6H_4$ | H | Cl | $CH_3$ | H | Harz | Z/E=3:2 |
| 6a | 4-F-$C_6H_4$ | 2-Cl-$C_6H_4$ | H | H | H | $CH_3$ | 1603,1509,1228 846,830,759 $cm^{-1}$ | Z |
| 7a | 4-F-$C_6H_4$ | 2-Cl-$C_6H_4$ | H | H | $CH_3$ | H | 93- 95°C | Z |
| 8a | 4-F-$C_6H_4$ | 2-Cl-$C_6H_4$ | Cl | Cl | Cl | H | 1603,1510,1470, 1233,847,753 $cm^{-1}$ | E |
| 9a | 4-F-$C_6H_4$ | 2-Cl-$C_6H_4$ | Cl | Cl | $CH_3$ | H | | |
| 10a | 4-F-$C_6H_4$ | 2-Cl-$C_6H_4$ | Br | Cl | Cl | H | 172-175°C | E |
| 11a | 4-F-$C_6H_4$ | 2-Cl-$C_6H_4$ | Br | Cl | $CH_3$ | H | | |
| 12a | 4-F-$C_6H_4$ | 4-Cl-$C_6H_4$ | H | Cl | Cl | H | | |
| 13a | 4-F-$C_6H_4$ | 4-Cl-$C_6H_4$ | Cl | Cl | Cl | H | | |
| 14a | 4-F-$C_6H_4$ | 4-Cl-$C_6H_4$ | Br | Cl | Cl | H | | |
| 15a | 4-F-$C_6H_4$ | 2,4-$Cl_2$-$C_6H_3$ | H | H | $CO_2CH_3$ | H | | |

Tabelle 2 - Forts.

| Bsp. | R1 | R2 | E | X | Y | Z | Schmp/IR | Isomer |
|---|---|---|---|---|---|---|---|---|
| 16a | 4-F-$C_6H_4$ | 2,4-$Cl_2$-$C_6H_3$ | Cl | H | $CO_2CH_3$ | H | | |
| 17a | 4-F-$C_6H_4$ | 2,4-$Cl_2$-$C_6H_3$ | Br | H | $CO_2CH_3$ | H | | |
| 18a | 4-F-$C_6H_4$ | 2-Cl-4-F-$C_6H_3$ | H | Cl | Cl | H | | |
| 19a | 4-F-$C_6H_4$ | 2-Cl-4-F-$C_6H_3$ | Cl | Cl | Cl | H | | |
| 20a | 4-F-$C_6H_4$ | 2-Cl-4-F-$C_6H_3$ | Br | Cl | Cl | H | | |
| 21a | 4-F-$C_6H_4$ | 2-$CF_3$-$C_6H_4$ | H | $NO_2$ | H | H | | |
| 22a | 4-F-$C_6H_4$ | 2-$CF_3$-$C_6H_4$ | H | H | $CO_2CH_3$ | H | | |
| 23a | 4-F-$C_6H_4$ | 2-$CF_3$-$C_6H_4$ | H | Cl | Cl | H | 1604, 1561, 1316, 1126, 768 $cm^{-1}$ | E/Z=2:1 |
| 24a | 4-F-$C_6H_4$ | 2-$CF_3$-$C_6H_4$ | H | H | CN | H | | |
| 25a | 4-F-$C_6H_4$ | 2-$CF_3$-$C_6H_4$ | H | Cl | $CH_3$ | H | 1510, 1316, 1167 1123, 769 $cm_{-1}$ | E/=2:1 |
| 26a | 4-F-$C_6H_4$ | 2-$CF_3$-$C_6H_4$ | H | H | H | $CH_3$ | | |
| 27a | 4-F-$C_6H_4$ | 2-$CF_3$-$C_6H_4$ | H | H | $CH_3$ | H | | |
| 28a | 4-F-$C_6H_4$ | 2-$CF_3$-$C_6H_4$ | Cl | Cl | Cl | H | | |
| 29a | 4-F-$C_6H_4$ | 2-$CF_3$-$C_6H_4$ | Cl | H | $CO_2CH_3$ | H | | |
| 30a | 4-F-$C_6H_4$ | 2-$CF_3$-$C_6H_4$ | Cl | Cl | $CH_3$ | H | | |
| 31a | 4-F-$C_6H_4$ | 2-$CF_3$-$C_6H_4$ | Br | Cl | Cl | H | | |
| 32a | 4-F-$C_6H_4$ | 2-$CF_3$-$C_6H_4$ | Br | Cl | $CH_3$ | H | | |
| 33a | 4-F-$C_6H_4$ | 2-$CF_3$-$C_6H_4$ | Br | H | $CO_2CH_3$ | H | | |
| 34a | 4-F-$C_6H_4$ | 4-$CF_3$-$C_6H_4$ | H | Cl | Cl | H | | |
| 35a | 4-F-$C_6H_4$ | 4-$CF_3$-$C_6H_4$ | Cl | H | $CO_2CH_3$ | H | | |
| 36a | 4-F-$C_6H_4$ | 4-$CF_3$-$C_6H_4$ | Br | Cl | $CH_3$ | H | | |
| 37a | 4-F-$C_6H_4$ | 2-F-$C_6H_4$ | H | Cl | Cl | H | | |
| 38a | 4-F-$C_6H_4$ | 2-F-$C_6H_4$ | H | Cl | $CH_3$ | H | | |
| 39a | 4-F-$C_6H_4$ | 2-F-$C_6H_4$ | Cl | Cl | Cl | H | | |

| Bsp. | $R^1$ | $R^2$ | E | X | Y | Z | Schmp/IR | Isomer |
|---|---|---|---|---|---|---|---|---|
| 40a | 4-F-$C_6H_4$ | 2-F-$C_6H_4$ | Cl | H | $CO_2CH_3$ | H | | |
| 41a | 4-F-$C_6H_4$ | 2-F-$C_6H_4$ | Br | Cl | Cl | H | | |
| 42a | 4-F-$C_6H_4$ | 2-F-$C_6H_4$ | Br | Cl | $CH_3$ | H | | |
| 43a | 4-F-$C_6H_4$ | 4-F-$C_6H_4$ | H | Cl | Cl | H | | |
| 44a | 4-F-$C_6H_4$ | 4-F-$C_6H_4$ | H | H | $CO_2CH_3$ | H | | |
| 45a | 4-F-$C_6H_4$ | 4-F-$C_6H_4$ | Cl | Cl | Cl | H | | |
| 46a | 4-F-$C_6H_4$ | 4-F-$C_6H_4$ | Cl | H | $CO_2CH_3$ | H | | |
| 47a | 4-F-$C_6H_4$ | 4-F-$C_6H_4$ | Br | Cl | Cl | H | | |
| 48a | 4-F-$C_6H_4$ | 4-Cl-$C_6H_4$ | Br | H | $CO_2CH_3$ | H | | |
| 49a | 4-F-$C_6H_4$ | 2,4-$F_2$-$C_6H_3$ | H | Cl | Cl | H | | |
| 50a | 4-F-$C_6H_4$ | 2,4-$F_2$-$C_6H_3$ | H | H | $CO_2CH_3$ | H | | |
| 51a | 4-F-$C_6H_4$ | 2,4-$F_2$-$C_6H_3$ | Cl | Cl | Cl | H | | |
| 52a | 4-F-$C_6H_4$ | 2,4-$F_2$-$C_6H_3$ | Cl | H | $CO_2CH_3$ | H | | |
| 53a | 4-F-$C_6H_4$ | 2,4-$F_2$-$C_6H_3$ | Br | Cl | Cl | H | | |
| 54a | 4-F-$C_6H_4$ | 2,4-$F_2$-$C_6H_3$ | Br | H | $CO_2CH_3$ | H | | |
| 55a | 4-F-$C_6H_4$ | 2,6-$F_2$-$C_6H_3$ | H | Cl | Cl | H | | |
| 56a | 4-F-$C_6H_4$ | 2,6-$F_2$-$C_6H_3$ | H | H | $CO_2CH_3$ | H | | |
| 57a | 4-F-$C_6H_4$ | 2,6-$F_2$-$C_6H_3$ | Cl | Cl | Cl | H | | |
| 58a | 4-F-$C_6H_4$ | 2,6-$F_2$-$C_6H_3$ | Cl | H | $CO_2CH_3$ | H | | |
| 59a | 4-F-$C_6H_4$ | 2,6-$F_2$-$C_6H_3$ | Br | Cl | Cl | H | | |
| 60a | 4-F-$C_6H_4$ | 2,6-$F_2$-$C_6H_3$ | Br | H | $CO_2CH_3$ | H | | |
| 61a | 4-F-$C_6H_4$ | 2-$OCH_3$-$C_6H_4$ | H | Cl | Cl | H | Harz | E/Z=3:1 |
| 62a | 4-F-$C_6H_4$ | 2-$OCH_3$-$C_6H_4$ | H | Cl | $CH_3$ | H | 1599, 1509, 1248, 1225, 754 $cm^{-1}$ | E/Z=3:1 |
| 63a | 4-F-$C_6H_4$ | 2-$OCH_3$-$C_6H_4$ | H | H | CN | H | Harz | E/Z=3:1 |

| Bsp. | $R^1$ | $R^2$ | E | X | Y | Z | Schmp/IR | Isomer |
|---|---|---|---|---|---|---|---|---|
| 64a | 4-F-$C_6H_4$ | 2-$OCH_3$-$C_6H_4$ | H | H | $CH_3$ | H | 1599, 1509, 1247, 754 $cm^{-1}$ | E/Z=3:1 |
| 65a | 4-F-$C_6H_4$ | 2-$OCH_3$-$C_6H_4$ | H | H | $CO_2CH_3$ | H | 1720, 1509, 1248, 1116, 755 $cm^{-1}$ | E/Z=3:1 |
| 66a | 4-F-$C_6H_4$ | 2-$OCH_3$-$C_6H_4$ | Cl | Cl | Cl | H | | |
| 67a | 4-F-$C_6H_4$ | 2-$OCH_3$-$C_6H_4$ | Br | H | $CO_2CH_3$ | H | | |
| 68a | 4-F-$C_6H_4$ | 4-$OCF_3$-$C_6H_4$ | H | Cl | Cl | H | | |
| 69a | 4-F-$C_6H_4$ | 4-$OCF_3$-$C_6H_4$ | Cl | Cl | Cl | H | | |
| 70a | 4-F-$C_6H_4$ | 4-$OCF_3$-$C_6H_4$ | Br | Cl | Cl | H | | |
| 71a | 4-F-$C_6H_4$ | $C_6H_5$ | H | Cl | Cl | H | | |
| 72a | 4-F-$C_6H_4$ | $C_6H_5$ | Cl | H | $CO_2CH_3$ | H | | |
| 73a | 4-F-$C_6H_4$ | Cyclohexyl | H | Cl | Cl | H | | |
| 74a | 4-F-$C_6H_4$ | Cyclohexyl | Cl | H | $CH_3$ | H | | |
| 75a | $C_6H_5$ | $C_6H_5$ | H | Cl | Cl | H | | |
| 76a | $C_6H_5$ | $C_6H_5$ | Cl | H | H | $CH_3$ | | |
| 77a | $C_6H_5$ | $C_6H_5$ | Br | H | $CO_2CH_3$ | H | | |
| 78a | $C_6H_5$ | 4-Cl-$C_6H_4$ | H | Cl | Cl | H | | |
| 79a | $C_6H_5$ | 2-Cl-$C_6H_4$ | Cl | Cl | Cl | H | | |
| 80a | $C_6H_5$ | 2,4-$Cl_2$-$C_6H_3$ | Br | H | $CO_2CH_3$ | H | | |
| 81a | $C_6H_5$ | 4-F-$C_6H_4$ | H | Cl | Cl | H | | |
| 82a | $C_6H_5$ | 2-F-$C_6H_4$ | H | Cl | Cl | H | | |
| 83a | 4-Cl-$C_6H_4$ | $C_6H_5$ | H | Cl | Cl | H | | |
| 84a | 4-Cl-$C_6H_4$ | 2-Cl-$C_6H_4$ | H | Cl | Cl | H | | |
| 85a | 4-Cl-$C_6H_4$ | 2-Cl-$C_6H_4$ | Cl | Cl | Cl | H | | |
| 86a | 4-Cl-$C_6H_4$ | 2,4-$Cl_2$-$C_6H_3$ | Br | H | $CH_3$ | H | | |
| 87a | 4-Cl-$C_6H_4$ | 4-F-$C_6H_4$ | H | Cl | Cl | H | | |

Tabelle 2 - Forts.

| Bsp. | $R^1$ | $R^2$ | E | X | Y | Z | Schmp/IR | Isomer |
|---|---|---|---|---|---|---|---|---|
| 88a | $4-Cl-C_6H_4$ | $2-F-C_6H_4$ | H | Cl | Cl | H | | |
| 89a | $2-Cl-C_6H_4$ | $2-Cl-C_6H_4$ | H | Cl | Cl | H | | |
| 90a | $2-Cl-C_6H_4$ | $2-F-C_6H_4$ | H | Cl | Cl | H | | |
| 91a | $2-F-C_6H_4$ | $2-Cl-C_6H_4$ | Cl | Cl | Cl | H | | |
| 92a | $2-F-C_6H_4$ | $2-F-C_6H_4$ | H | Cl | Cl | H | | |
| 93a | $2-F-C_6H_4$ | $4-F-C_6H_4$ | Br | Cl | Cl | H | | |
| 94a | $2-F-C_6H_4$ | $4-F-C_6H_4$ | H | Cl | Cl | H | | |
| 95a | Cyclohexyl | $2-Cl-C_6H_4$ | H | Cl | Cl | H | | |
| 96a | Cyclohexyl | $4-Cl-C_6H_4$ | Cl | Cl | Cl | H | | |
| 97a | Cyclohexyl | $4-F-C_6H_4$ | H | Cl | Cl | H | | |
| 98a | $4-F-C_6H_4$ | $2-Cl-C_6H_4$ | F | $CH_3$ | Cl | H | | |
| 99a | $4-F-C_6H_4$ | $2-Cl-C_6H_4$ | F | Cl | Cl | H | | |
| 100a | $4-F-C_6H_4$ | $4-Cl-C_6H_4$ | F | H | CN | H | | |
| 101a | $4-F-C_6H_4$ | $2,4-Cl_2-C_6H_3$ | F | H | CN | H | | |
| 102a | $4-F-C_6H_4$ | $2-Cl-4-F-C_6H_3$ | F | Cl | Cl | H | | |
| 103a | $4-F-C_6H_4$ | $2-CF_3-C_6H_4$ | F | $CH_3$ | Cl | H | | |
| 104a | $4-F-C_6H_4$ | $4-CF_3-C_6H_4$ | F | Cl | Cl | H | | |
| 105a | $4-F-C_6H_4$ | $2-F-C_6H_4$ | F | H | $CH_3$ | H | | |
| 106a | $4-F-C_6H_4$ | $4-F-C_6H_4$ | F | Cl | Cl | H | | |
| 107a | $4-F-C_6H_4$ | $2,4-F_2-C_6H_3$ | F | H | $CO_2CH_3$ | H | | |
| 108a | $4-F-C_6H_4$ | $2-OCH_3-C_6H_4$ | F | H | $CO_2CH_3$ | H | | |
| 109a | $4-F-C_6H_4$ | $4-OCF_3-C_6H_4$ | F | Cl | Cl | H | | |
| 110a | $C_6H_5$ | $4-Cl-C_6H_4$ | F | Cl | Cl | H | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosphorium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mitteln enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotiil.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 113 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 115 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 113 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 115 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 113 werden mit 3 Gew.-Teilen des Natriumsalzes der Diisobutylnaphthalin-α-sulfonsäure, 10 Gew.-Teilen des Natriumsalzes einer Ligninsulfonsäure aus eine Sulfitablauge und 7 Gew.-Teilen pulverförmigen Kieselsäuregel gut vermischt und in einer Hammermühle vermahlen. Durch feines Verteilen der Mischung in Wasser erhält man einer Spritzbrühe.

VI. 3 Gew.-Teile der Verbindung Nr. 115 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 113 werden mit einer Mischung aus 92 Gew.-Teilen pulverförmigen Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 115 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 113 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,

2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiel

Als Vergleichswirkstoff wurde cis-2-(imidazol-1-yl-methyl)-2-(4-fluorphenyl)-3-(2,6-difluorphenyl)-oxiran (A) - bekannt aus DE 3 218 130 -benutzt.

Anwendungsbeispiel

Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Est dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten. Das Ergebnis zeigt, daß die Wirkstoffe 113 und 115 bei der Anwendung als 0,05 %ige (Gew.%) Spritzbrühe eine bessere fungizide Wirkung zeigen (95 %) als der bekannte Vergleichswirkstoff A (60 %).

## Ansprüche

1. Substituierte Imidazolylmethyloxirane und substituierte Imidazolylpropene der allgemeinen Formel I

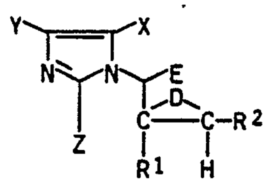

I

in welcher
$R^1$ und $R^2$ gleich oder verschieden sind und $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4-C-Atomen substituiert sein können,
D den Rest 0 oder eine Einfachbindung bedeutet,
E den Rest H, F, Cl oder Br bedeutet,
X, Y und Z gleich oder verschieden sind und Wasserstoff, Halogen, Nitro, Cyano, $C_1$-$C_3$-Alkyl, $C_1$-$C_3$-Halogenalkyl, $C_1$-$C_3$-Alkoxy, Amino, gegebenenfalls durch $C_1$-$C_3$-Alkyl, $C_1$-$C_3$-Acyl substituiertes Amino, Mercapto, gegebenenfalls durch $C_1$-$C_3$-Alkyl substituiertes Mercapto, $C_1$-$C_3$-Acyl, $C_1$-$C_3$-Alkoxycarbonyl, $C_1$-$C_3$-Hydroxyalkyl oder gegebenenfalls substituiertes Phenyl bedeuten, wobei X, Y und Z nicht gleichzeitig Wasserstoff bedeuten,
sowie deren für Pflanzen verträgliche Säureadditionssalze und Metallkomplexe.

2. Substituierte Imidazolylmethyloxirane und substituierte Imidazolylpropene der allgemeinen Formel I, in denen $R^1$ und $R^2$ die Phenylgruppe bedeutet, welche unsubstituierte oder durch einen oder zwei Substituenten substituiert ist, die Fluor, Chlor, Brom oder die Trifluormethylgruppe bedeuten.

3. Verfahren zur Herstellung der substituierten Imidazolylmethyloxirane der Formel I gemäß Anspruch 1, in der D der Rest O und E den Rest H bedeutet, dadurch gekennzeichnet, daß man

a) eine Verbindung der Formel II

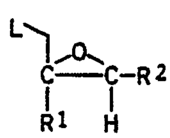

II

in welcher $R^1$ und $R^2$ die oben angegebenen Bedeutungen haben und L eine nukleophil substituierbare Abgangsgruppe darstellt, mit einer Verbindung der Formel III

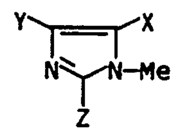

III

in der Me ein Wasserstoffatom, ein Metallatom oder eine Trimethylsilylgruppe bedeutet und X, Y und Z die oben angegebene Bedeutung haben, umsetzt oder

b) eine Verbindung der Formel IV

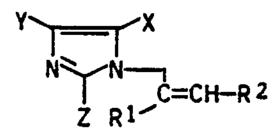

IV

in welcher $R^1$, $R^2$, X, Y und Z die oben angegebene Bedeutung haben, in das entsprechende Oxiran überführt und die so erhaltenen Verbindungen gegebenenfalls in ihre Salze mit für Pflanzen verträglichen Säuren überführt.

4. Verfahren zur Herstellung der substituierten Imidazolylpropene der Formel I gemäß Anspruch 1, in der D den Rest - und und E den Rest H bedeutet, dadurch gekennzeichnet, daß man

a) eine Verbindung der Formel V

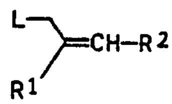

V

in der $R^1$, $R^2$ und L die oben angegebenen Bedeutung haben, mit einer Verbindung der Formel III

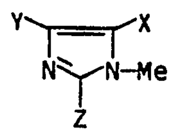

III

in der X, Y, Z und Me die oben angegebene Bedeutngen haben, umsetzt oder

b) eine Verbindung der Formel VI

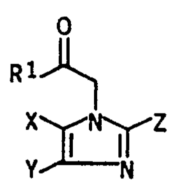

VI

in welcher $R^1$, X, Y und Z die oben angegebene Bedeutung hat, mit einer Verbindung der Formel VII

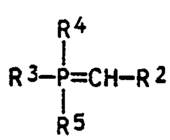

VII

in der $R^2$ die oben angegebene Bedeutung hat und $R^3$, $R^4$ und $R^5$, die gleich oder verschieden sein können, den Phenylrest, den p-Carboxyphenylrest, den p-Dimethylaminophenylrest, die Dimethylamino, Piperidino- oder Morpholinogruppe, Alkylreste mit 1 bis 3-C-Atomen oder die Cyclohexylgruppe darstellen, umsetzt.

5. Verfahren zur Herstellung der substituierten Imidazolylmethyloxirane und der substituierten Imidazolylpropene der Formel I gemäß Anspruch 1, in der E den Rest F, Cl oder Br bedeutet, dadurch

gekennzeichnet, daß man eine Verbindung der Formel VIII

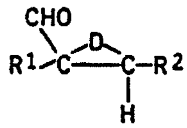

VIII

in welcher $R^1$, $R^2$ und D die angegebenen Bedeutungen haben, mit einer Verbindung der Formel III

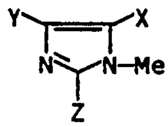

III

in der X, Y, Z und Me die oben angegebenen Bedeutungen haben, in Gegenwart von Thionylhalogeniden umsetzt.

6. Fungizides Mittel, enthaltend einen Trägerstoff und eine fungizid wirksame Menge eines Imidazolylmethyloxirans der Formel I

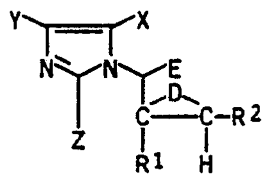

I

in welcher
$R^1$ und $R^2$ gleich oder verschieden sind und $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4-C-Atomen substituiert sein können,
D den Rest 0 oder oder eine Einfachbindung bedeutet,
E den Rest H, F, Cl oder Br bedeutet,
X, Y and Z gleich oder verschieden sind und Wasserstoff, Halogen, Nitro, Cyano, $C_1$-$C_3$-Alkyl, $C_1$-$C_3$-Halogenalkyl, $C_1$-$C_3$-Alkoxy, Amino, gegebenenfalls durch $C_1$-$C_3$-Alkyl, $C_1$-$C_3$-Acyl substituiertes Amino, Mercapto, gegebenenfalls durch $C_1$-$C_3$-Alkyl substituiertes Mercapto, $C_1$-$C_3$-Acyl, $C_1$-$C_3$-Alkoxycarbonyl, $C_1$-$C_3$-Hydroxyalkyl oder gegebenenfalls substituiertes Phenyl bedeuten, wobei X, Y und Z nicht gleichzeitig Wasserstoff bedeuten,
oder dessen für Pflanzen verträglichen Säureadditionssalzes oder Metallkomplexes.

7. Verfahren zur Bekämpfung von Pilzen, dadurch gekennzeichnet, daß man eine fungizid wirksame Menge eines substituierten Imidazolylmethyloxirans oder eines substuierten Imidazolylpropens der Formel I

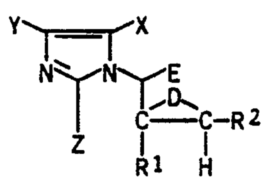

I

in welcher
$R^1$ und $R^2$ gleich oder verschieden sind und $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4-C-Atomen substituiert sein können,
D den Rest 0 oder eine Einfachbindung bedeutet,
E den Rest H, F, Cl oder Br bedeutet,

X, Y und Z gleich oder verschieden sind und Wasserstoff, Halogen, Nitro, Cyano, $C_1$-$C_3$-Alkyl, $C_1$-$C_3$-Halogenalkyl, $C_1$-$C_3$-Alkoxy, Amino, gegebenenfalls durch $C_1$-$C_3$-Alkyl, $C_1$-$C_3$-Acyl substituiertes Amino, Mercapto, gegebenenfalls durch $C_1$-$C_3$-Alkyl substituiertes Mercapto, $C_1$-$C_3$-Acyl, $C_1$-$C_3$-Alkoxycarbonyl, $C_1$-$C_3$-Hydroxyalkyl oder gegebenenfalls substituiertes Phenyl bedeuten, wobei X, Y und Z nicht gleichzeitig Wasserstoff bedeuten,
oder dessen für Pflanzen verträgliches Säureadditionssalz oder Metallkomplex auf die Pilze oder durch Pilzbefall bedrohte Materialien, Flächen, Pflanzen oder Saatgüter einwirken läßt.

8. Verbindung der Formel I gemäß Anspruch 1, dadurch gekennzeichnet, daß $R^1$ 4-Fluorphenyl, $R^2$ 2,6-Difluorphenyl, D den Rest O, E den Rest H, X den Rest H, Z den Rest H und Y Methoxycarbonyl bedeuten.

9. Verbindung der Formel I gemäß Anspruch 1, dadurch gekennzeichnet, daß $R^1$ 4-Fluorphenyl, $R^2$ 2,6-Difluorphenyl, D den Rest O, E den Rest H, Z den Rest H, X den Rest H und Y Acetyl bedeuten.

10. Verbindung der Formel I gemäß Anspruch 1, dadurch gekennzeichnet, daß $R^1$ 4-Fluorphenyl, $R^2$ 2-Chlorphenyl, D den Rest O, E den Rest Chlor, Z den Rest Methoxycarbonyl und X und Y Wasserstoff bedeuten.